Europäisches Patentamt

European Patent Office

Office européen des brevets

(19)

(11) Publication number : **0 494 724 A2**

# EUROPEAN PATENT APPLICATION

(12)

(21) Application number : **92250003.8**

(22) Date of filing : **06.01.92**

(51) Int. Cl.⁵ : **C12N 15/82, A01H 5/00**

A request for correction (on page 13, plasmid pat 2 Hy Triple-X, deposit nr. DSM 6865 of example 4 was not mentioned) beenfiled pursuant to Rule 88 EPC. A decision on the request will be taken during the proceedings before the Examining Division(Guidelines for Examination in the EPO, A-V, 2.2).

The microorganisms have been deposited with Deutsche Sammlung von Microorganismen (DSM) under numbers DSM 6280 and DSM 6281.

(30) Priority : **08.01.91 DE 4100594**

(43) Date of publication of application :
**15.07.92 Bulletin 92/29**

(84) Designated Contracting States :
**AT BE CH DE DK ES FR GB GR IT LI LU NL SE**

(71) Applicant : **INSTITUT FÜR GENBIOLOGISCHE FORSCHUNG BERLIN GMBH**
**Ihnestr.63**
**W-1000 Berlin 33 (DE)**

(72) Inventor : **Gatz, Christiane, Dr.**
**Assmannshauser Strasse 10A**
**W-1000 Berlin 33 (DE)**
Inventor : **Frohberg, Claus**
**Moltkestrasse 31A**
**W-1000 Berlin 45 (DE)**
Inventor : **Kaiser, Astrid**
**Bismarckstrasse 3a**
**W-1000 Berlin 37 (DE)**

(54) **Plasmids for controlling expression in plants.**

㊼   Plasmids that comprise DNA sequences that permit the expression, controlled in respect of time and location, of a heterologous product in plants are described. The expression is not achieved until an inducer has been added.
   The DNA sequences include a functional combination of plant expression sequences and bacterial control sequences.
   Plants that comprise the DNA sequences of those plasmids are also described. Tetracycline is used as an inducer.

EP 0 494 724 A2

The present invention relates to plasmids that contain DNA sequences that permit the control of gene expression in plants.

The properties of plants can be improved by inserting new genetic information into the plant cell nucleus. That genetic information is contained in DNA sequences which, when inserted into the plant, result in the production of heterologous products in the plant. The DNA sequences described here mediate that those products are only being formed until a low-molecular-weight substance has been added.

Heterologous products expressed by the DNA sequences may be, for example, substances such as proteins that confer resistance to pests and chemicals, enzymes that intervene in the metabolism of the plant, proteins that are ussed in diagnostics and medicine, or ribonucleic acids that inhibit the expression of endogenous products.

The expression of those or other gene products can be controlled by the use of suitable control sequences. The control sequences may be isolated from the genome of the plant itself. Control sequences are already known the activity of which is controlled by parameters such as light (Kuhlemeier et al., Ann. Rev. Plant Physiol. (1987), 38, 221-257), temperature (Ainley and Key, Plant Mol. Biol. (1990), 14, 949-966), anaerobiosis (Freeling and Bennett, Ann. Rev. Genet. (1985) 19, 297-332), damage (Keil et al., EMBO J. (1989), 8, 1323-1330), plant hormones (Guilfoyle, CRC (1985) 247-276), and salicylic acid (Mol et al., EP 337532), or which are active only in certain organs, such as, for example, leaves (Stockhaus et al., EMBO J. (1989), 8, 2445-2451), blossoms (Goldberg, Science 1988, 240, 1460-1467) or roots (Lam et al., Proc. Natl. Acad. Sci. USA (1989), 86, 7890-7894).

The present invention provides a plasmid that comprises means for controlling, in respect of time and location, the expression of a DNA sequence in a plant. The DNA sequence is generally a sequence that codes for a heterologous product, although, if desired, the sequence may code for a product that is naturally present in the chosen plant host.

In a plasmid of the invention, the control means is generally such that expression of the DNA sequence is effected only in the presence of an inducer, and so generally comprises an operator and a gene for a repressor. Preferred control means are those with which the inducer interacts specifically, that is to say, the inducer interacts only with the newly constructed control system and has substantially no effect on other control systems in the genome of the plant in which the control means is to be inserted.

The present invention, for example, provides plasmids that comprise a functional combination of plant expression control sequences and bacterial control sequences such that the expression, controlled in respect of time and location, of a heterologous product can be achieved by the controlled addition of an inducer, the inducer interacting only with the newly constructed control system and having no appreciable effect on other control sequences present in the genome.

This type of combination of bacterial control sequences with control elements for gene expression from encaryotic organism can also be established in any other organism such as mice, human cell lines, flies and yeast.

The present invention also provides plants that comprises the DNA sequences of plasmids of the invention.

In contrast to known combinations of naturally occuring regulatable plant control sequences, plasmids according to the invention may comprise a functional combination of a plant expression control sequences and one or more bacterial control elements, for example, repressor-operator-inducer systems. In such a system a repressor protein binds with a high degree of affinity to a specific operator sequence. If that operator sequence is in the promotor region of a gene, the expression of that gene is prevented. A repressor interacts with a specific inducer molecule which causes the repressor protein to becom detached from the DNA and which thereby induces gene activity. Bacterial control systems are available for that purpose, the activity of which is controlled by active substances such as antibiotics (Hillen et al., J. Mol. Biol. (1983), 172, 185-), sugar derivatives or amino acid derivatives (Pabo and Sauer, Ann. Rev. Biochem. (1984), 53, 293-321 or aromatic compounds (Nermod et al., J. Bact. (1986), 167, 447-454).

The present invention provides a plasmid which comprises a plant expression control sequence and one or more operator sequences, for example, of a bacterial control sequence. A plasmid of the invention may comprise a plant promoter in which has been inserted one or more operator sequences The arrangement of the plant promoter, the oprator(s) and the DNA coding for the desired product should be such that the DNA sequence is expressed only in the presence of the inducer for the repressor.

The combination of plant expression sequences, for example, promotors and bacterial control elements or other operators according to the present invention results in a parcularly high degree of specificity and good controllability when the following prerequisites are met: the inducer does not occur in the plant, is absorbed well, is not metabolised or otehwise modified or inactivated, and has no appreciable effect on endogenous plant proteins. An inducer which interacts with bacterial control elements in a transgenic plant has the advantage over substances which interact with plant control elements that only the expression of a newly incorporated product is affected and not the expression of a series of gene products present naturally in the genome of the plant.

Using initial means of the invention, expression of the desired product in the presence of a repressor does not take place until an inducer is supplied to the plant, the expression taking place only in those organs which are treated with the inducer.

As indicated above, the expression of a heterologous product in transgenic plants becomes controllable by the insertion of operator sequences into a plant promoter such that a repressor protein binds to those operator sequences and thereby prevents expression, the repressor protein losing its ability to bind to the operator sequences as a result of the addition of an inducer, which, as a result, leads to the expression of the heterologous product. The operator sequences therefore may be of prokaryotic or eukaryotic origin.

It has also been found that the combination of bacterial operator sequences and a plant promotor is particularly effective if two operator sequences are located 3' of the TATA element of a plant promoter.

Behind the plant promoter there is preferably a polylinker which permits the insertion of various gene sequences, and also a termination region.

It has also been found that the regulation elements of the tetracycline restistance operon, for example, of transposon Tn10, are suitable for controlling the activity of a plant promoter.

The repressor protein therefor is a polypeptide 207 amino acids long that binds with a high degree of affinity ($K_{eq} = 10^{-11}$M under physiological saline conditions) to a 19 bp operator sequence (Kleinschmidt et al., Biochemistry (1988), 27, 1094-1104).

The high equilibrium constant of a $10^{-9}$M of the repressor-tetracycline complex permits an efficient detachment of the repressor from the DNA even at very low tetracycline concentations (Takahashi et al., J. Mol. Biol. (1986), 187, 341-348).

Tetracycline, a derivative of tetracycline, or another compound that, by binding to the tet repressor, is capable of bringing about a change in the latters's DNA binding property, can be used as an inducer for such a system.

Tetracycline itself has proved to be an especially effective inducer, because like most antibiotics, readily diffuses through membranes and can accordingly be absorbed by plant cells. In plants there are no tetracycline-like active substances that inactivate the repressor from the start.

It has been found to be particularly advantageous to provide two tet operators, especially located 3' of the TATA element of the plant promoter. It is even more advantageous to locate one operator upstream of the TATA-Box in addition to the two operators downstream.

In the control means of the present invention it is preferable to use a strong promoter, for example, the cauliflower mosaic virus promoter. A promoter may have enhancer elements, for example, a cauliflower mosaic virus (CaMV) 35S promoter may have CaMV 35S promoter enhancer elements, which bring about constitutive expression, or may have other enhancer elements that confer a different expression pattern.

An example of control means according to the present invention comprises a cauliflower mosaic virus 35S promoter fragment having enhancer elements, a TATA box, two or three operators and a DNA sequence coding for a heterologous product.

The cauliflower mosaic virus 35S promoter fragment having enhancer elements, for example, CaMV 35S promoter enhancer elements is, for example, a 334 bp fragment, and the TATA box and two tet operators are, for example, present together with a linked CCC fragment, in a 99 bp oligo-DNA fragment having the following sequence:

```
       1         10        20        30        40        50        60        70
       |         |         |         |         |         |         |         |
    CCCACTAGTCTTCGCAAGACCCTTTACGTATATAAGGAGATCTCTATCACTGATAGGGAGTGTTAACATAA
    GGGTGATCAGAAGCGTTCTGGGAAATGCATATATTCCTCTAGAGATAGTGACTATCCCTCACAATTGTATT

              80        90       100
              |         |         |
    CTCTATCACTGATAGAGTGATCCTTTCTAGA
    GAGATAGTGACTATCTCACTAGGAAAGATCT
```

The cauliflower mosaic virus 35S promoter fragment having the opertor sites in the vicinity of the TATA

3

box, one operator being located 5′ of the TATA box and 2 operators 3′ of the TATA box, together with a 83 bp DNA fragment having the following sequence:

```
1              10            20            30            40
CTAGACTCTATCAGTGATAGAGTGTATATAAGACTCTATCAGTGA
     TGAGATAGTCACTATCTCACATATATTCTGAGATAGTCACT

    50            60            70            80
TAGAGTGAACTCTATCAGTGATACAGTTAACGGTACCT
ATCTCACTTGAGATAGTCACTATGTCAATTGCCATGGAGATC
```

A control system may comprise one or more further elements, for example, any one or more of the following: a polylinker sequence, a polyadenylation signal, for example, a 203 bp polyadenylation signal of the nopaline synthase gene, and selectable marker gene, for example, an antibiotica restistance gene, for example, a 1650 bp hygromycin phosphotransferase gene.

By way of example, a control system may comprise a polylinker sequence, a 334 bp cauliflower mosaic virus 35S promoter fragment having enhancer elements, for example CaMV 35S promoter enhancer elements, a 99 bp oligo-DNA fragment having a TATA box and two tet operators, a DNA sequence coding for a heterologous product, a 203 bp polyadenylation signal of the nopaline synthase gene and a 1650 bp hygromycin phosphotransferase gene, the 99 bp oligo DNA fragment preferably having the sequence given above.

A large number of cloning vectors comprising a replication system in E. coli and a marker that permits selection of the transformed cells are available for preparation for the insertion of foreign genes into higher plants. The vectors comprise, for example, pBR 322, pUC series, M13 mp series, pACYC 184, etc.. Accordingly, the desired sequence can be inserted into the vector at a suitable restriction site. The resulting plasmid is used for transformation into E. coli. The E. coli cells are cultivated in a suitable nutrient medium, then harvested and lysed. The plasmid is recovered. Sequence analysis, restriction analysis, electrophoreses and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted.

The use of a T-DNA for the transformation of plant cells has been intensively researched and described, for example, in EP 120 516; Hoekema, In: The Binary Plant Vector System Offset-drukkerij Kanters B.V., Alblasserdam, 1985, Chapter V; Fraley et al, Crit. Rev. Plant Sci., 4: 1-46 and An et al., EMBO J. (1985) 4: 277-287.

Once the inserted DNA has been integrated in the genome it is relatively stable there and, as a rule, does not come out again. It normally comprises a selection marker that confers on the transformed plant cells resistance to a biocide or an antibiotic, such as kanamycin, G 418, bleomycin, hygromycin or chloramphenicol, inter alia. The individually employed marker should accordingly permit the selection of transformed cells rather than cells that do not contain the inserted DNA.

A large number of techniques are available for inserting DNA into a plant host cell. Those techniques include transformation with T-DNA using Agrobacterium tumefaciens or Agrobacterium rhizogenes as transformation agent, fusion, injection or electroporation as well as other possible methods. If agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in agrobacteria. The intermediate vector can be transferred into Agrobacterium tumefaciens by means of a helper plasmid (conjugation). Binary vectors can replicate themselves both in E. coli and in agrobacteria. They comprise a selection marker gene and a linker or polylinker which are framed by the right and left T-DNA border regions. They can be transformed directly into agrobacteria (Holsters et al., Mol. Gen. Genet. (1978), 163: 181-187). The agrobacterium used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with <u>Agrobacterium</u> <u>tumefaciens</u> or <u>Agrobacterium</u> <u>rhizogenes</u> for the transfer of the DNA into the plant cell. Whole plants can then be regenerated from the infected plant material (for example pieces of leaf, serpents of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. The plants can be grown in the normal manner and crossed with plants that have the same transformed hereditary factors or other hereditary factors. The resulting hybrid individuals have the corresponding phenotypic properties.

It will be appreciated that the control system of the present invention will function in a plant only if the appropriate repressor protein is present, for example, when the control means comprises a tet operator, a tet repressor must be provided. Accordingly, if the host plant does not already produce the appropriate repressor, it is necessary to provide the plant with means for producing the repressor, that is to say, DNA coding for the repressor.

In general, that may be achieved by cloning the gene, or an appropriate gene fragment, for the repressor in a suitable vector and inserting the gene in a plant. In the construction of a suitable vector, appropriate plant expression control elements should be provided in order to ensure expression of the repressor in the host plant. It is preferable , for example, to use a strong, for example, a constitutive plant promoter.

Suitable techniques for inserting DNA into a plant host cell are described above.

The present invention provides a plasmid that comprises DNA coding for a repressor, for example, for a tet repressor, and also provides plants that comprise the control means of the present invention and also comprise DNA coding for the appropriate repressor.

The present invention further provides the use of a plasmid comprising control means of the present invention that includes an operator and, generally, also the use of a plasmid that comprises DNA coding for the appropriate repressor in the production of a plant in which expression of a DNA sequence is controllable in time and in location.

The control in respect of time and location of expression of a desired product in a transgenic plant of the present invention is achieved by the addition of the inducer for the control system that has been inserted into the plant: expression will take place only when the inducer is present, and will take place only in those locations where the inducer is present.

Control in respect of time may be achieved, for example, by the timing, frequency and number of applications of the inducer.

It will be appreciated that for use with a transgenic plant of the present invention, an inducer should be cabable of reaching the repressor, that is to say, the transgenic plant must be able to take up the inducer and, once inside the plant, the inducer must be able to reach the repressor. Furthermore, the inducer should not be toxic to the plant.

As indicated above, tetracycline fulfils the above criteria. Other suitable inducers include tetracycline derivatives that bind to the repressor and abolish its DNA binding capacity. The appropriate opprator/repressor system for a chosen inducer may be incorporated in a plant as described herein, for example, in analogy to the tetracycline operator/repressor system.

**Terms and abbreviations**

**Abbreviations**

bp, kb =        base pairs, kilobases

DNA =          deoxyribonucleic acid, carrier of genetic information

RNA =          ribonucleic acid

HEPES =      N-2-hydroxyethylpiperazine-N′-2-ethane-sulphonic acid

kDa =          kilodalton

SDS =          sodium dodecyl sulphate

tris =          tris(2-aminoethyl)amine

EDTA =        ethylenediaminetetraacetic acid

mmol =        millimole

fmol =        femtomole

The following plasmids were deposited at the Deutsche Sammlung von Mikroorganismen (DSM) in Brunswick, Federal Republic of Germany on 23.12.1990 (deposit number):

plasmid pTET1 (DSM 6281)
plasmid pAT2HyStu4 (DSM 6280)

**Description of the Figures**

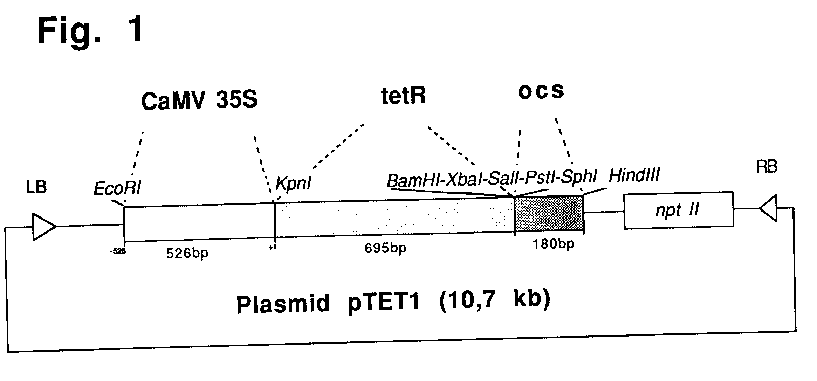

Figure 1 shows the restriction map of the 10.7 kb plasmid pTET1. The abbreviations have the following meanings:

LB =        left border sequence of T-DNA

RB =        right border sequence of T-DNA

CaMV 35S =  promoter (526 bp) of cauliflower mosaic virus

tetR =       structural gene (695 bp) of the tetra-cycline repressor

ocs =       polyadenylation signal of the octopine synthase gene (180 bp)

nptII =     chimaeric gene of neomycin phospho-transferase under the control of the nopaline synthase promoter (1200 bp)

The cleavage sites described in Example 1 are also shown.

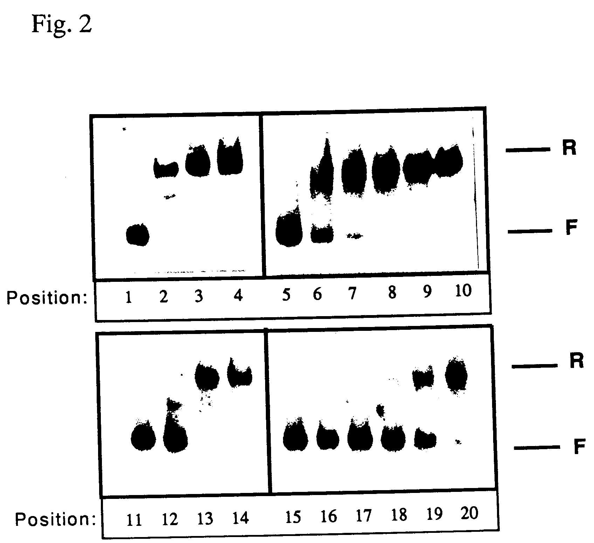

Figure 2 shows the autoradiogram of an electrophoresis for detecting the functional tet repressor in transgenic tobacco plants. Inserted at positions 1 to 10 were 6 fmol and, at positions 11 to 20, 20 fmol of $^{32}$P-labelled operator fragment (360 bp). In positions 1 to 4, the binding reaction was carried out in the presence of 0 (control), 20, 60 and 120 fmol of tet repressor (purified from Escherichia coli). In positions 5 to 10, the binding reaction was carried out in the presence of 0 (control), 1, 2, 4, 6 and μg of protein extract from leaves of a transgenic plant comprising the T-DNA of plasmid pTET1; in positions 11 to 14, the binding reaction was carried out in the presence of 0 (control), 10, 100 and 200 fmol of tet repressor (purified from Escherichia coli); in positions 15 to 20, the binding reaction was carried out in the presence of 0 (control), 1, 2, 4, 8 and 16 μg of protein extract from protoplasts of a transgenic plant comprising the T-DNA of plasmid pTET1.

The abbreviations have the following meanings:

R =    tet repressor-DNA complex

F =    non-complexed DNA

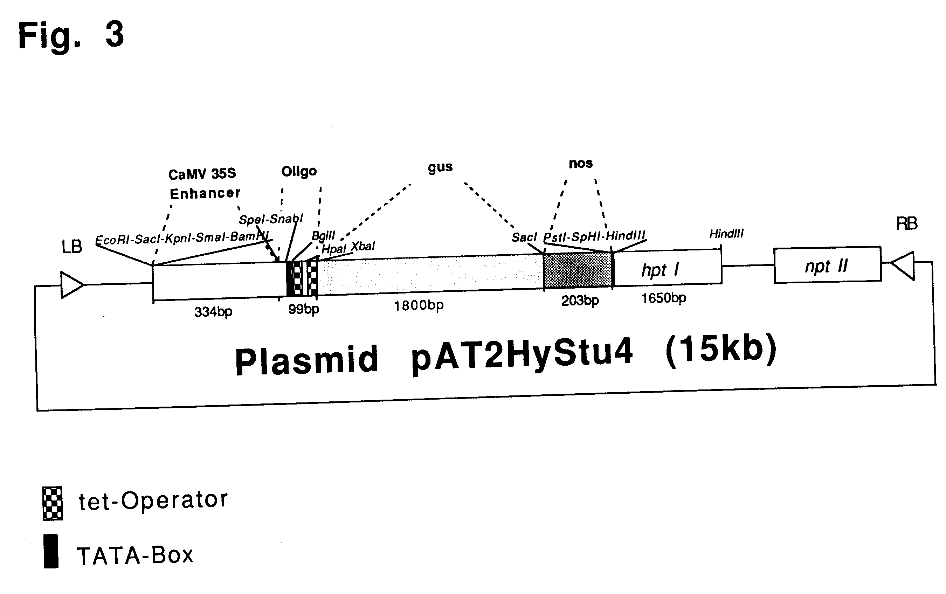

Figure 3 shows the restriction map of the 15 kb plasmid pAT2HyStu4. The abbreviations have the following meanings:

LB =        left border sequence of T-DNA

RB =        right border sequence of T-DNA

CaMV 35S =  promoter (enhancer) (334 bp) of cauliflower mosaic virus comprising two tet operators

oligo =     DNA fragment (99 bp) comprising the TATA box, two tet operators and the cleavage sites Spel, Snabl, BglII, Hpal and Xbal

gus =      structural gene (1800 bp) of β-glucuronidase from Escherichia coli

nos =      polyadenylation signal of the nopaline synthase gene (203 bp)

hptI =      chimaeric gene of hygromycin phospho-transferase (1650 bp) under the control of the nopaline synthase promoter

nptII =     chimaeric gene of neomycin phospho-transferase under the control of the nopaline synthase promoter (1200 bp)

The cleavage sites described in Example 2 are also shown.

Figure 4 shows the autoradiogram of the Northern blot analysis for detecting the tetracycline-dependent expression of the gus gene in extracts from leaves of 6 independent transgenic plants (positions 1 to 6) that comprise the T-DNA of plasmid pTET1 and of plasmid pAT2HyStu.

+ =    RNA from leaves of transgenic plants after infiltration with tetracycline

- =    RNA from leaves of transgenic plants without infiltration with tetracycline

Figure 5 shows the autoradiogram of the Northern blot analysis for characterising the concentration dependence of tetracycline induction in extracts from leaves of a transgenic plant (positions 1 to 5) after infiltration with different concentrations of tetracycline (Tc (mg/l): 0.1, 0.5, 1 and 10) and a control (Tc (mg/l) : 0).

The abbreviations have the following meanings:

Tc =    tetracycline

gus =    mRNA of β-glucuronidase from Escherichia coli

tetR =    mRNA of the tet repressor

Figure 6 shows the gel of the Northern blot analysis for characterising the development in time of tetracycline induction in extracts from leaves of a transgenic plant (positions 1 to 6). Samples were taken after 0 (control), 0.5, 1, 3, 6 and 22 hours (h). The abbreviations have the following meanings:

gus = mRNA of β-glucuronidase from Escherichia coli

tetR = mRNA of the tet repressor

Figure 7 shows the local induction of Gus-expression in a leave of a transgenic plant.

In order to understand the Examples forming the basis of this invention, all the processes that are necessary for these tests and which are known per se will first of all be listed:

1. Cloning process

Vectors pUC18/19 (Yanisch-Perron et al., Gene (1985), 33, 103-119) were used for cloning.

For plant transformation, the gene constructions were cloned into the binary vector BIN19 (Bevan, Nucl. Acids Res. (1984), 12, 8711-8720). Standard methods were carried out in accordance with known procedures (Molecular Cloning, Maniatis et al., (1982), Cold Spring Harbor Laboratory Press, Cold Spring Harbor, N.Y.).

2. Bacterial strains

The E. coli strain DH 5 Alpha of genotype F⁻, end A1, hsdR17, (r$\bar{\text{k}}$, m$_k$+), supE44, thi-I, R⁻, recA1, gyrA96, rdA1, ∅ 80d laczΔ15 was used for the pUC vectors.

The plant transformation was carried out by means of the Agrobacterium tumefaciens strain GV 2260 Deblaere et al., Nucl. Acids Res. 13, 4777-4788, (1985).

3. Transformation of Agrobacterium tumefaciens

The insertion of the DNA into the agrobacteria was effected by direct transformation in accordance with the method developed by Holsters et al., (Mol. Gen. Genet. (1978), 163, 181-187). The plasmid DNA of transformed agrobacteria was isolated in accordance with the method developed by Birnboim and Doly (Nucl. Acids Res. (1979), 7, 1513-1523) and was separated by gel electrophoresis after suitable restriction cleavage.

4. Plant transformation

10 ml of an overnight culture of Agrobacterium tumefaciens in YEB medium, consisting of 0.5 % beef extract, 0.1 % yeast extract, 0.5 % peptone, 0.5 % sucrose and 2 mM magnesium sulphate, were centrifuged off, the supernatant was discarded and the bacteria were resuspended in the same volume of antibiotic-free medium. In a sterile Petri dish, leaf discs of sterile plants (approximately 1 cm$^2$), the central vein of which had been removed, were immersed in that bacterial suspension. The leaf discs were then placed in a closely packed arrangement in Petri dishes containing MS medium (according to Murashige and Skoog, Physiologia Plantarum (1962), 15, 473-497) with 2 % sucrose and 0.8 % Bacto agar. After two days incubation in the dark at 25°C, they were transferred onto MS medium containing 100 mg/l of kanamycin or hygromycin, 500 mg/l of claforan, 1 mg/l of benzylaminopurine (BAP), 0.2 mg/l of naphthylacetic acid (NAA) and 0.8 % Bacto agar. Growing shoots were transferred onto hormone-free MS medium with 250 mg/l of claforan and 50 mg/l of kanamycin or hygromycin.

Shoots that formed roots on that medium were tested by Northern and Southern blot analysis for the integration of the DNA sequences to be investigated.

5. Analysis of genomic DNA from transgenic plants

The isolation of genomic plant DNA was effected in accordance with Rogers and Bendich (Plant Mol. Biol. (1985), 5, 69-76).

For the DNA analysis, after suitable restriction cleavage 20 μg of DNA were analysed by means of Southern blotting for the integration of the DNA sequences to be investigated.

The DNA, in SSC buffer containing 3M sodium chloride and 0.3M sodium citrate, was blotted onto a membrane. Hybridisation was effected in the hybridisation buffer according to Amasino (Anal. Biochem. (1986), 152, 304-307) containing 10 % polyethylene glycol. In order to detect the integrated DNA sequence, the DNA fragment was radioactively labelled.

6. Analysis of the total RNA from transgenic plants

The isolation of plant total RNA was carried out in accordance with Logemann et al. (Analytical Biochem. (1987), 163, 16-20).

For the analysis, 30 μg portions of total RNA were investigated by means of Northern blotting for the presence of the transcripts sought. Blotting and hybridisation were carried out in accordance with the same procedure as given for the Southern blot analysis.

8. Detection of the tet repressor in transgenic plants

100 mg of leaf material from plants comprising the T-DNA of plasmid pTET1 were homogenised, without freezing, in 200 μl of extraction buffer (50 mM sodium dihydrogen phosphate/disodium hydrogen phosphate pH 7,5, 1 mM EDTA, 0.1 % 4-(1,1,3,3-tetramethylbutyl)phenol, 10 mM β-mercaptoethanol, 2 mM sodium hydrogen sulphite, 2 mM phenylmethylsulphonyl fluoride, 1 μg/ml antipain, 1 μg/ml aprotinin, 1 μg/mm chymostatin, 1 μg/ml leupeptin, 1 μg/ml pepstatin and 0.1 g polyvinylpyrrolidone/g leaf material).

The binding reaction was carried out in 40 μl of tris buffer (50 mM sodium chloride, 10 mM magnesium chloride and 10 mM tris/HCl, pH 7.5) there being added to the buffer from 5 to 20 fool of $^{32}$P-end-labelled operator fragment having a specific activity of 0.6 Ci $^{32}$P/mmol, and 80 μg of herring sperm DNA. From 0.5 to 10 μl of leaf extract were then added to that binding reaction mixture. After a period of incubation of 15 minutes at room temperature, sufficient glycerol was added to obtain a 25 % solution. The solution was added to a 5 % polyacrylamide gel and separated by gel electrophoresis. The electrophoresis was carried out in tris buffer pH 8.0 (60 mM tris/base, 60 mM boric acid, 1 mM EDTA and 10 % glycerol) for 12 hours at 2.5 V/cm. The gels were then dried. The radio-active bands that occurred were rendered visible by autoradiography.

9. Protoplast preparation

Protoplasts from leaves of axenic plants comprising the T-DNA of plasmid pTET1 were prepared in accordance with the method developed by Damm and Willmitzer (Mol. Gen. Genet. (1988), 213, 15-20). The protoplasts were counted, concentrated by centrifugation and taken up in 100 μl of extraction buffer, 300,000 protoplasts being contained in 100 μl of buffer. The protoplasts lysed in that buffer. The cell detritus was then centrifuged off.

10. Assays for Gus activity

For the fluorometric Gus assay explants were homogenized and incubated with the substrate 4-methylumbelliferyl-β-D glucuronide at 37 °C. Quantification of the fourescence was done according to Jefferson (Plant Mol. Biol. Rep. 5, 387-405, 1987). Protein concentrations were determined according to Bradford (1979). For in vivo staining intact plan material was vacuum infiltrated with 1mM X-Gluc (5-bromo-4-chloro-3-indolyl-β-D glucuronic acid cyclohexylammonium) and incubated overnight at 37 °C.

## Example 1

Preparation of plasmid pTET1 and insertion of the T-DNA of the plasmid into the plant genome of tobacco

The two plasmids BINAR (Höfgen and Willmitzer, Plant Science (1990), 66, 221 - 230) and pWH 305 (Ohemichen et al., EMBO J. (1984), 3, 539 - 543) were used for the preparation of plasmid pTET1. The BINAR plasmid is a derivative of the binary vector BIN19 (Bevan, Nucl. Acids Res. (1984), 12, 8711 - 8720) which derivative contains between the EcoRI and the HindIII site of the polylinker the promoter of the 35S RNA of cauliflower mosaic virus (CaMV), which promoter brings about a constitutive expression, and also the polyadenylation signal of the octopine synthase gene. Located between the promoter and the polyadenylation signal are the following cleavage sites: KpnI, SmaI, BamHI, XbaI, SalI, PstI. The 695 bp HpaI fragment from pWH305 was inserted into the SmaI cleavage site. The HpaI fragment contains 18 bp of the 5'-untranslated leader, 624 bp of the structural gene and 51 bp of the 3'-region of the tetR gene. The SmaI cleavage site was lost during the cloning process. Plasmid pTET1 has a total size of 10.7 kb which is composed as follows: between the EcoRI site and the KpnI site is the 526 bp promoter fragment of the CaMV 35S virus (Covey and Hull (1985) Advances in Cauliflower Mosaic Virus Research, Oxford Surveys of Plant Molecular and Cell Biology, 2, 339 - 346); between the KpnI site and the BamHI site is the 695 bp fragment of the tetR gene; between the BamHI and the SphI site is a polylinker sequence having the above-mentioned cleavage sites; between the SphI site and the HindIII site is the 180 bp polyadenylation signal of the octopine synthase gene. As soon as it has been integrated in the plant genome, that portion of the plasmid brings about the synthesis of the tet repressor protein in all tissues. The sequences outside the EcoRI site and the HindIII site are sequences of vector BIN19. They comprise all functions for the replication and selection of the plasmid in Escherichia coli and Agrobacterium tumefaciens as well as for the transfer of T-DNA into plants and the potential for selecting transformed plant cells on kanamycin. Resistance is conferred by the nptII gene. That plasmid was transformed into Agrobacterium tumefaciens. DNA transfer into tobacco plants was then carried out by co-cultivation of those agrobacteria with small pieces of leaf from tobacco plants. The selection was carried out on kanamycin. Intact and fertile plants were regenerated from transformed cells.

Ten independently transformed plants were examined for the synthesis of mRNA for the tet repressor by Northern blot analysis.

As is already known, the amounts of mRNA in independent transgenic plants varies considerably (Sanders et al., Nucl. Acids Res. (1987), 15, 1543 - 1558). A plant having the highest yield of synthesised mRNA was used for all further analyses.

In order to quantify the amount of functional tet repressor protein in that plant, a gel retard analysis was carried out in accordance with Fried and Crothers (Nucl. Acids Res. (1981), 9, 6505 - 6525) (see Figure 2). That method of analysis exploits the fact that a protein-DNA complex runs more slowly during electrophoresis on a 5 % polyacrylamide gel (see band R in Figure 2) than does the unbound DNA (see band F in Figure 2). From 1 to 8 μg of protein of the crude extract from leaves of one of the plants, which synthesises the transcript for the synthesis of the tet repressor, were incubated with 6 fmol of a purified $^{32}$P-end-labelled operator fragment and subjected to electrophoresis on a 5 % polyacrylamide gel. As shown in Figure 2, the mobility of the fragment in positions 5 to 10 is retarded to the same degree as in positions 2 to 4, where repressor protein purified from E. coli was used in the binding reaction. That protein was obtained in accordance with the purification process according to Ohemichen et al. (EMBO J. (1984), 3, 539 -543). Those positions serve as controls to indicate that the operator binding protein occurring in plant extracts has the same electrophoretic mobility as the tet repressor. It can accordingly be assumed that the plant containing the tetR transcript synthesises functional tet repressor. No retardation was observed with the extract from untransformed tobacco plants. The concentration of the operator fragment in the binding reaction is 0.25 x 10$^{-9}$M. The binding constant of the repressor to operator DNA, under the saline conditions used here (50 mM sodium chloride, 10 mM magnesium chloride) is > 0.5 x 10$^{-11}$M (Kleinschmidt et al., Biochemistry (1988), 27, 1094-1104). Accordingly, in order to calculate the amount of repressor, it can be assumed that quantitative binding takes place under those conditions. Since the binding of four monomers each having a molecular weight of 24 kDa results in a complete retardation of the fragment, it was calculated, on the basis of track 8, that an amount of 24 fmol of tet repressor is contained in 4 μg of protein extract, which constitutes 0.01 % of the total amount of protein. In order to ascertain the number of tet repressors per cell, protoplasts of the same tobacco plant were prepared. Positions 15 to 20 in Figure 2 show a titration experiment in which extracts of 5000, 10,000, 20,000, 40,000 and 80,000 protoplasts were used. 20 fmol of DNA fragment were used for the experiment. Approximately 18 fmol were retarded in position 20, which indicates 72 fmol of repressor in 80,000 protoplasts. Since 72 fmol of repressor are equivalent to 4.5 x 10$^{10}$ repressor molecules, it can be assumed that there is a rate of synthesis of at least 500,000 repressor molecules per cell in the analysed plant.

## Example 2

Preparation of plasmid pAT2HyStu4 and stable integration of an operator-containing CaMV 35S promoter into a tobacco plant that synthesises the tet repressor

It was the aim of the following cloning steps to combine two tet operator sequences, to which the tet repressor binds, with the CaMV 35S promoter. Like most other eukaryotic promoters that are recognised by polymerase II, the CaMV 35S promoter comprises a TATA box, in the vicinity of which general transcription factors are active, and, in addition, 5′-positioned enhancer elements which, in the case of the CaMV 35S promoter, are responsible for a strong constitutive expression in all tissues of the plant. The operator sequences were located in the immediate vicinity of the TATA box so that the tet repressor binding thereto interferes with the general transcription factors active in this region. This strategy has the advantage that the newly created DNA sequence, namely the operator sequences combined with the TATA box, can also be combined with other enhancer elements which confer a different expression pattern from that conferred by the enhancer elements of the CaMV 35S promoter.

Since in the wild-type sequence of the promoter there is no suitable cleavage site for the insertion of an additional DNA fragment, the sequence from base pairs -56 to +7 (+1 corresponds to the transcription start site) was altered as follows: analogously to the cloning process already described (Gatz and Quail, Proc. Natl. Acad. Sci. USA (1988), 85, 1394 - 1397) the wild-type sequences of the promoter in the region from -56 to +7 were replaced by a synthetic DNA fragment having the following primary structure:

```
-56                                                              +1
CCCACTAGTCTTCGCAAGACCCTTTACGTATATAAGGCCTTTCTAGACATTTGCTCGA
      ATCAGAAGCGTTCAGGGAAATGCATATATTCCGGAAAGCTCTGTAAACGAGCTCTAG
```

That synthetic DNA fragment has the following cleavage sites:

SpeI:    ACTAGT from positions -53 to -48
SnabI:    TACGTA from positions -32 to -27

Stul:     AGGCCT from positions -22 to -17

Xbal:     TCTAGA from positions -15 to -10

Xhol:     CTCGAG from positions + 3 to - 3

Blglll:    AGATCT from positions + 2 to + 7

and the sequence TATATAA, which is important for the promoter activity, from positions - 31 to - 24 .

As a result of that cloning, the CaMV 35S promoter was changed to the extent that it was possible to insert DNA fragments into the cleavage sites listed here. The changes do not affect the promoter activity. This promoter is called heeeafter caMV (Res.).

A 55 bp synthetic sequence was inserted into the Stul site. The primary structure of that synthetic DNA is as follows:

```
AGATCTCTATCACTGATAGGGAGAGTTAACATAACTCTATCACTGATAGAGTGAT
TCTAGAGATAGTGACTATCCCTCTCAATTGTATTGAGATAGTGACTATCTCACTA
```

The Stul site was lost during cloning.

After that cloning step, the sequence of the operator-containing CaMV 35S promoter from positions -56 to +61 is as follows:

```
 1        10        20        30        40        50        60        70
 |         |         |         |         |         |         |         |
CCCACTAGTCTTCGCAAGACCCTTTACGTATATAAGGAGATCTCTATCACTGATAGGGAGTGTTAACATAA
GGGTGATCAGAAGCGTTCTGGGAAATGCATATATTCCTCTAGAGATAGTGACTATCCCTCACAATTGTATT
-56 (Position 1)
```

```
         80        90        100       110
          |         |         |         |
CTCTATCACTGATAGAGTGATCCTTTCTAGACATTTGCTCGAGATCT
GAGATAGTGACTATCTCACTAGGAAAGATCTGTAAACGAGCTCTAGT
                                        +61
                                        (Position 118)
```

That sequence comprises the following structural features:

Spel:          ACTAGT from positions 4 to 9

Snabl:         TACGTA from positions 25 to 30

TATA box:      TATATAA from positions 29 to 35

Blglll:        AGATCT from positions 38 to 43

tet operator:  TCTCTATCACTGATAGGG from positions 41 to 59

Hpal:          GTTAAC from positions 62 to 67

tet operator:  ACTCTATCACTGATAGAGT from positions 71 to 89

Xbal:          TCTAGA from positions 97 to 102

Xhol:          CTCGAG from positions 109 to 114

Blglll:        AGATCT from positions 113 to 118

As a result of the combination of that sequence with a plant enhancer element, a promoter is formed which is not read in the plant in the presence of the tet repressor. In the presence of the tetracycline inducer, however, the promoter is active.

In the Examples shown here, the 118 bp DNA fragment is present in combination with the enhancer elements of the CaMV 35S promoter (see above for cloning strategy).

A fragment a total of 471 bp long, which contains the base pairs -390 to -56 of the wild-type CaMV 35S promoter and also the synthetic sequence including the XbaI site (positions 97 to 102), was cloned in the form of an EcoRI/XbaI fragment into the vector pGUS (cleaved with EcoRI and XbaI). That vector pGUS comprises the sequence of the structural gene for bacterial β-glucuronidase (gus) (Jefferson et al., EMBO J. (1987), 6, 3901 - 3907) in front of which is located a polylinker having cleavage sites for EcoRI, SacI, KpnI, SmaI, BamHI and XbaI. The polyadenylation signal of the nopaline synthase gene is positioned at the 3′-end. That chimaeric gene was cut out in the form of an EcoRI/HindIII fragment and cloned between the EcoRI/HindIII site of BIN19 (Bevan, Nucl. Acids Res. (1984) 12, 8711 - 8720). A chimaeric hygromycin phosphotransferase gene was then cloned into the HindIII site of that BIN19 derivative under the control of the nopaline synthase promoter so that it was possible to select the transformation event in the already kanamycin-resistant plants that comprised the T-DNA of plasmid pTEt1. Instead of the hygromycin phosphotransferase gene it is also possible to use another resistance gene, the only exception being the neomycin phosphotransferase gene. The restriction map of the recombinant DNA pAT2HyStu4 is shown in Figure 3. The Figure is intended to illustrate that the 15 kb plasmid pAT2HyStu4 is composed of the following fragments: between the cleavage sites EcoRI and BamHI is a sequence of a polylinker; between BamHI and SpeI is the 334 bp DNA fragment that comprises the enhancer elements of the CaMV 35S promoter as well as the sequence CCC; between SpeI and XbaI are 99 bp of the synthetic oligo-DNA fragment that comprises a TATA box and two tet operators.

The base sequence of that 99 bp synthetic oligo-DNA fragment and of the CCC sequence is as follows:

```
1           10          20          30          40          50          60          70
|           |           |           |           |           |           |           |
CCCACTAGTCTTCGCAAGACCCTTTACGTATATAAGGAGATCTCTATCACTGATAGGGAGTGTTAACATAA
GGGTGATCAGAAGCGTTCTGGGAAATGCATATATTCCTCTAGAGATAGTGACTATCCCTCACAATTGTATT
```

```
            80          90          100
            |           |           |
CTCTATCACTGATAGAGTGATCCTTTCTAGA
GAGATAGTGACTATCTCACTAGGAAAGATCT
```

Between XbaI and SacI is the structural gene (1800 bp) of β-glucuronidase; between SacI and HindIII is the polyadenylation signal of the nopaline synthase gene (203 bp); between two HindIII sites is the hygromycin phosphotransferase gene under the control of the nopaline synthase promoter (1650 bp). The sequences inside the EcoRI site and the second HindIII site comprise the information for the synthesis of β-glucuronidase and also the information for expressing resistance to hygromycin. The sequences outside the EcoRI site and the second HindIII site are sequences of vector BIN19. They comprise all functions for the replication and selection of the plasmid in Escherichia coli and Agrobacterium tumefaciens as well as for the transfer of the T-DNA into plants and the potential for selecting transformed plant cells on kanamycin. The resistance is conferred by the nptII gene or the hptI gene.

That plasmid was transformed into Agrobacterium tumefaciens. DNA transfer into tobacco plants was then carried out by co-cultivation of those agrobacteria with small pieces of leaf from tobacco plants. Tobacco plants that comprise the T-DNA of plasmid pTET1 and therefore synthesise the tet repressor were used for those transformations. The synthesis of β-glucuronidase in dependence on the addition of tetracycline takes place only in the presence of the tet repressor. The selection was carried out on hygromycin. Intact and fertile plants were regenerated from transformed cells.

## Example 3

Detection of tetracycline-dependent expression of the β-glucuronidase gene from pAT2HyStu4 in leaves of transformed tobacco plants synthesising the tet repressor

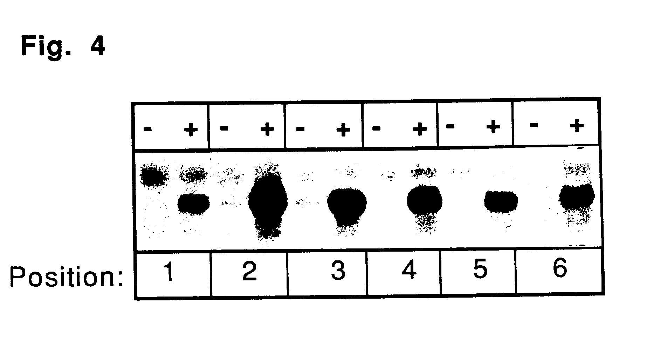

In order to bring about homogeneous absorption of the tetracycline inducer in al cells, the antibiotic was introduced into the intercellular space by infiltrating individual leaves with a tetracycline-containing buffer. For that purpose, individual leaves of six independent transformed plants were placed in a beaker with 50 mM sodium citrate buffer containing 10 mg/l of tetracycline. The beaker was placed in a desiccator and maintained under vacuum for 3 minutes. As a result of the vacuum, air escaped from the intercellular space. That air was replaced by the buffer during the ventilation of the desiccator. After infiltration, the leaves were incubated for 16 hours on MS medium with 10 mg/l of tetracycline. Control leaves were treated in exactly the same manner but without the addition of tetracycline. The RNA of the leaves was then prepared, applied to a 1 % agarose gel and subjected to Northern blot analysis, the transcript of the gus mRNA being made visible by hybridisation with a radioactively labelled fragment from the structural gene of the β-glucuronidase. The RNA analysis shown in Figure 4 demonstrates that the mRNA that comprises the sequences of the gene for β-glucuronidase is formed in the plants comprising the T-DNAs of plasmids pTET1 and pAT2HyStu4 only if the leaves have been pretreated with tetracycline.

In order to determine the smallest amount of tetracycline required for induction, leaves of a plant that comprised the T-DNAs of plasmids pTET1 and pAT2HyStu4 were infiltrated with 50 mM sodium citrate buffer containing different concentrations of tetracycline.

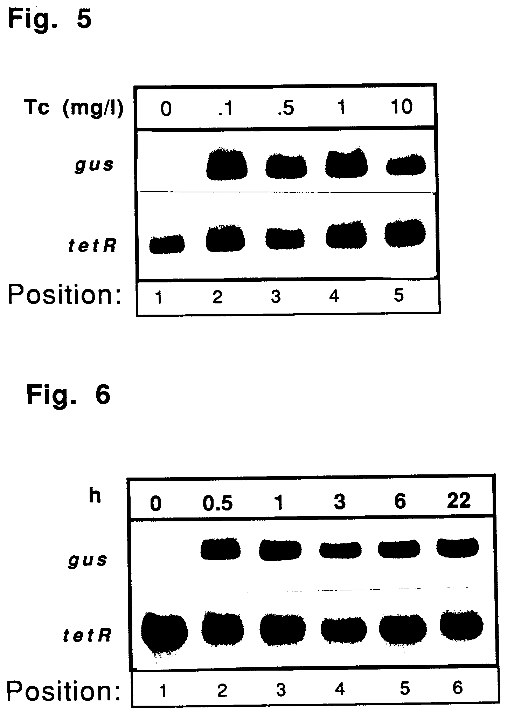

The leaves were infiltrated with 0 (control), 0.5, 1.0 and 10.0 mg/l, respectively, of tetracycline in 50 mM sodium citrate buffer and placed on MS medium containing an equivalent tetracycline concentration in each case. Northern blot analysis shows that even 0.1 mg/l of tetracycline is sufficient for maximum induction (see Figure 5, position 2).

In order to investigate the time-dependence of the induction of the gus mRNA, leaves of a plant comprising the T-DNAs of plasmids pTET1 and pAT2HyStu4 were infiltrated with 50 mM sodium citrate buffer containing 10 mg/l of tetracycline and placed on MS medium for 0, 0.5, 1, 3, 6 and 22 hours, respectively. RNA extraction and Northern blot analysis were then carried out. The addition of 10 mg/l of tetracycline resulted in complete induction of the gus mRNA after only half an hour (see Figure 6, position 2).

Figures 5 and 6 at the same time show that the RNA samples contain the mRNA for the gene of the tet repressor. The mRNA is already visible in position 1 in each Figure, since its expression takes place constitutively.

Any desired gene may by cloned according to the cloning strategy described in Example 2, for example, any desired gene may be substituted for the β-glucuronidase gene in the process described in Example 2. After transformation into Agrobacrerium tumefaciens and transfer into a plant that is capable of producing tet repressor, the desired product will be produced only in the presence of tetracycline.

## Example 4

Preperation of plasmid pAT2HyTriple-X

It was the aim of the following cloning steps to improve the repression efficiency of the regulatory system described above. It has been proposed by Lin and Riggs (Cell 4, 107-111, 1975) that repression efficiencies increase with the number of operators within a promoter, if each copy by itself contributes to repression. Therefore we have constructed another CaMV 35S promoter fragment which contains three operator sites in the vicinity of the TATA-box, one operator being located 5′ of the TATA-box and 2 operators 3′ of the TATA-box.

This was achieved by inserting a 83 bp synthetic sequence into the plasmid described above (page 25), which contains the CaMV 35S promotor derivative CaMV 35S (Res.). The 56 bp fragment between Spel and Xhol (positions are indicated on page 25 ), were exchanged by a snythetic 83 bp DNA fragment containing the following sequence:

```
         1         10          20          30          40
         I          I           I           I           I
      CTAGACTCTATCAGTGATAGAGTGTATATAAGACTCTATCAGTGA
           TGAGATAGTCACTATCTCACATATATTCTGAGATAGTCACT


            50          60          70          80
             I           I           I           I
        TAGAGTGAACTCTATCAGTGATACAGTTAACGGTACCT
        ATCTCACTTGAGATAGTCACTATGTCAATTGCCATGGAGATC
```

That sequence comprises the following structural features.

| | | |
|---|---|---|
| TATA-box: | TATATAA | from position 25 to 31 |
| tet operator 1: | ACTCTATCAGTGATAGAGT | from position 5 to 23 |
| tet operator 2: | | from position 33 to 51 |
| tet operator 3: | | from position 53 to 71 |
| HpaI: | GTTAAC | from position 71 to 76 |
| KpnI: | GGTACC | from position 77 to 82 |

The resulting altered CaMV 35S promoter derivative is called hereafter CaMV 35S (TripleX). Cloning of the CaMV 35S TripleX-promoter as an EcoRI/Xbal fragment into the vector pGus and subsequent introduction of this gene in transgenic plants which synthesize the repressor protein was done as described above.

## Example 5

Detection of tetracycline-dependent expression of the β-glucuronidase gene from pAT2HyTriple-X in leaves of transformed tobacco plants synthesizing the tet repressor.

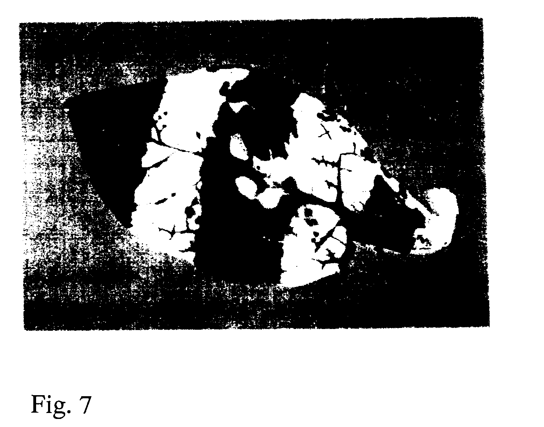

The plants containing DNA sequences encoding the information for synthesis of the tet repressor and the CaMV 35S (Triple-X) promoter regulating the expression of the β-glucuronidase gene were analysed for tetracycline dependent gene expression. Tetracycline is efficiently taken up through the roots, which leads to homogeneous expression of the gene under the control of the 3 operator containing CaMV 35S promoter. Based on the level of enzyme activity, the activity in the absence of the inducer was 500 fold lower than in the presence of the inducer. The gene product of the reporter gene β-Glucuronidase was only expressed in plants which had been treated with Tetracycline. Local induction can also be achieved by applying tetracycline directly on leaves. This is demonstrated in Figure 7.

## Claims

1. A plasmid that comprises means for controlling the time and the location of the expression of a DNA sequence in a plant.

2. A plasmid as claimed in claim 1, wherein the DNA sequence codes for a heterologous product.

3. A plasmid as claimed in claim 1 or claim 2, wherein the control means is such that expression of the DNA sequence is effected only in the presence of an inducer.

4. A plasmid as claimed in any one of claims 1 to 3, wherein the control means comprises a functional combination of a plant control sequence and one or more operators.

EP 0 494 724 A2

5. A plasmid as claimed in claim 4, wherein the control means comprises a plant promoter in which has been inserted one or more operator sequences, one or more genes for a repressor, the arrangement of the plant promoter, the operator(s) and the DNA sequence being such that the DNA sequence is expressed only in the presence of the inducer for the repressor.

6. A plasmid as claimed in claim 4 or claim 5, wherein the or each operator sequence is of prokaryotic or eukaryotic origin.

7. A plasmid as claimed in any one of claims 3 to 6, wherein the or each operator comprises the regulatory elements of a tetracycline resistance operon.

8. A plasmid as claimed in claim 7, wherein the operator comprises the regulatory elements of the tetracycline resistance operon of transposon Tn10.

9. A plasmid as claimed in any one of claims 3 to 8, wherein two operator sequences are located 3′ of the TATA element of the plant promoter.

10. A plasmid as claimed in any one of claims 1 to 9, which also comprises one or more of the following: a polylinker sequence, a polyadenylation signal and a DNA sequence coding for a selectable marker.

11. A plasmid as claimed in claim 9 or claim 10, which comprises a cauliflower mosaic virus 355 promoter having enhancer elements, a TATA box, two or three tet operators and a DNA sequence coding for a heterologous product.

12. A plasmid as claim in claim 11, which comprises a polylinker sequence, a 334 bp cauliflower mosaic virus 35S promoter fragment having enhancer elements, a 99 bp oligo-DNA fragment having a TATA-box and two tet opertors, a DNA sequence coding for a heterologous product, a 203 bp polyadenylation signal of the nopaline synthase gene and a 1650 bp hygromycin phosphotransferase gene.

13. A plasmid as claimed in claim 11 or claim 12, wherein the TATA box and the two tet operators are present, together with a linked CCC fragment, in a 99 bp oligo DNA fragment having the following sequence:

```
    1        10        20        30        40        50        60        70
    |         |         |         |         |         |         |         |
CCCACTAGTCTTCGCAAGACCCTTTACGTATATAAGGAGATCTCTATCACTGATAGGGAGTGTTAACATAA
GGGTGATCAGAAGCGTTCTGGGAAATGCATATATTCCTCTAGAGATAGTGACTATCCCTCACAATTGTATT


            80        90       100
             |         |         |
CTCTATCACTGATAGAGTGATCCTTTCTAGA
GAGATAGTGACTATCTCACTAGGAAAGATCT
```

14. A plasmid as claimed in claim 11, wherein the TATA box and the three operators are present, together with a 83 bp DNA fragment having the following sequence:

14

```
        1           10           20           30           40
        |           |            |            |            |
      CTAGACTCTATCAGTGATAGAGTGTATATAAGACTCTATCAGTGA
           TGAGATAGTCACTATCTCACATATATTCTGAGATAGTCACT


            50           60           70           80
            |            |            |            |
         TAGAGTGAACTCTATCAGTGATACAGTTAACGGTACCT
         ATCTCACTTGAGATAGTCACTATGTCAATTGCCATGGAGATC
```

15. Plasmid pAT2HyStu4 (DSM 6280).

16. Plasmid pAT2HyTriple-X (DSM      ).

17. A plasmid that comprises a 526 bp cauliflower mosaic virus 35S promoter fragment, a 695 bp tet repressor fragment, and a 180 bp polyadenylation signal of the octopine synthase gene.

18. Plasmid pTET1 (DSM 6281).

19. A plant that comprises means for controlling the time and the location of the expression of a DNA sequence.

20. A plant as claimed in claim 19, wherein the DNA sequence codes for a heterologous product.

21. A plant as claimed in claim 19 or claim 20, wherein the control means is as defined in any one of claims 3 to 10 or comprises the control elements defined in any one of claims 11 to 13.

22. A plant as claimed in any one of claims 19 to 21, which comprises a plasmid as claimed in any one of claims 1 to 15.

23. A plant as claimed in any one of claims 19 to 22, which also comprises a DNA sequence that codes for the repressor for the operator present.

24. A plant as claime in any one of claims 19 to 23, wherein the operator is a tet operator and the repressor is a tet repressor protein.

25. A plant as claimed in claim 24, which comprises a plasmid as claimed in claim 17 or claim 18.

26. A plant which comprises plasmid pTET1 (DSM 6281), plasmid pAT2HyStu4 (DSM 6280) and plasmid pAT2HyTriple-X (DSM      ).

27. A plant as claimed in any one of claims 19 to 26, which is a tobacco plant.

28. Use of a plasmid as claimed in any ony of claims 1 to 14 in production of plants in which expression of a specific DNA sequence is controllable in time and in location.

29. Use of a plasmid as claimed in any one of claims 1 to 14 and a plasmid coding for the corresponding repressor for the production of plants in which expression of a specific DNA sequence is controllable in time and in location

30. Use of plasmids pTET1, pAT2HyStu4 and pAT2Hy Triple-X for the production of plants in which expression of a specific DNA sequence is controllable in time and in location.

EP 0 494 724 A2

# Fig. 1

Plasmid pTET1 (10,7 kb)

Fig. 2

# Fig. 3

Plasmid pAT2HyStu4 (15kb)

▦ tet-Operator

▮ TATA-Box

EP 0 494 724 A2

# Fig. 4

| - | + | - | + | - | + | - | + | - | + | - | + |

Position: | 1 | 2 | 3 | 4 | 5 | 6 |

# Fig. 5

| Tc (mg/l) | 0 | .1 | .5 | 1 | 10 |
|---|---|---|---|---|---|
| gus | | | | | |
| tetR | | | | | |
| Position: | 1 | 2 | 3 | 4 | 5 |

# Fig. 6

| h | 0 | 0.5 | 1 | 3 | 6 | 22 |
|---|---|---|---|---|---|---|
| gus | | | | | | |
| tetR | | | | | | |
| Position: | 1 | 2 | 3 | 4 | 5 | 6 |

Fig. 7